(19) Europäisches Patentamt

European Patent Office

Office européen des brevets

(11) **EP 1 559 725 A1**

(12) **EUROPEAN PATENT APPLICATION**
published in accordance with Art. 158(3) EPC

(43) Date of publication:
**03.08.2005 Bulletin 2005/31**

(51) Int Cl.[7]: **C07K 14/705**, C07K 16/28,
A61K 9/127, A61K 39/395,
A61K 51/00, A61P 35/00,
C12N 15/09

(21) Application number: **03751332.2**

(22) Date of filing: **03.10.2003**

(86) International application number:
**PCT/JP2003/012732**

(87) International publication number:
**WO 2004/089984 (21.10.2004 Gazette 2004/43)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **04.10.2002 JP 2002291953**

(71) Applicant: **Mitsubishi Pharma Corporation
Osaka-shi, Osaka 541-0046 (JP)**

(72) Inventors:
• **HIRAKAWA, Youko,
c/o MITSUBISHI PHARMA CORPORATION
Tokyo 103-8405 (JP)**
• **NIKI, Hisae,
c/o MITSUBISHI PHARMA CORPORATION
Tokyo 103-8405 (JP)**

• **OIKE, Shinsuke,
c/o MITSUBISHI PHARMA CORPORATION
Tokyo 103-8405 (JP)**
• **TAGAWA, Toshiaki,
MITSUBISHI PHARMA CORPORATION
Tokyo 103-8405 (JP)**
• **HOSOKAWA, Saiko,
c/o MITSUBISHI PHARMA CORPORATION
Tokyo 103-8405 (JP)**
• **YOSHIYAMA, Yoshiko,
c/o ZOEGENE CORPORATION
Yokohama-shi, Kanagawa 227-8502 (JP)**

(74) Representative:
**TER MEER STEINMEISTER & PARTNER GbR
Patentanwälte,
Mauerkircherstrasse 45
81679 München (DE)**

(54) **ANTIGEN RECOGNIZING ANTIBODY**

(57) An antigen having a part which is exposed on the surface of a cell at the formation of a tumor mass, and a pharmaceutical composition and a labeling agent comprising a ligand which recognizes the antigen.

EP 1 559 725 A1

SEQUENCE LISTING

<110> MITSUBISHI PHARMA CORPORATION

<120> Antibody recognition antigen

<130> 03038W00

<150> JP 2002-291953
<151> 2002-10-04

<160> 22

<170> PatentIn version 3.1

<210> 1
<211> 9
<212> PRT
<213> Homo sapiens

<220>
<223> Inventor:Hirakawa, Youko; Niki, Hisae; Oike Shinsuke
      Inventor:Tagawa, Toshiaki; Hosokawa, Saiko; Yoshiyama, Yoshiko

<400> 1
Ile Ser Ser Cys Gly Phe Tyr Trp Asn
1               5

<210> 2
<211> 12
<212> PRT
<213> Homo sapiens

<400> 2
Ile Gly Tyr Ile Tyr Tyr Ser Gly Ser Thr Tyr Tyr
1               5                   10

<210> 3
<211> 9
<212> PRT
<213> Homo sapiens

<400> 3
Ser Thr Arg Leu Arg Gly Ala Asp Tyr
1               5

<210> 4
<211> 17
<212> PRT
<213> Homo sapiens

<400> 4
Lys Ser Ser Gln Ser Val Leu Tyr Asn Ser Asn Asn Lys Lys Tyr Leu
1               5                   10                  15
Ala

<210> 5
<211> 7
<212> PRT
<213> Homo sapiens

<400> 5
Trp Ala Ser Thr Arg Glu Ser
1               5

<210> 6

<211> 9
<212> PRT
<213> Homo sapiens

<400> 6
Gln Gln Tyr Tyr Ser Thr Pro Trp Thr
1               5

<210> 7
<211> 119
<212> PRT
<213> Homo sapiens

<400> 7
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Cys
                20                  25                  30
Gly Phe Tyr Trp Asn Trp Ile Arg Gln His Pro Gly Lys Gly Leu Glu
                35                  40                  45
Trp Ile Gly Tyr Ile Tyr Tyr Ser Gly Ser Thr Tyr Tyr Asn Pro Ser
        50                  55                  60
Leu Lys Ser Arg Val Thr Ile Ser Leu Asp Thr Ser Lys Ser Gln Phe
65                  70                  75                  80
Ser Leu Lys Leu Ser Ser Leu Thr Ala Ala Asp Thr Ala Val Tyr Tyr
                85                  90                  95
Cys Ala Arg Ser Thr Arg Leu Arg Gly Ala Asp Tyr Trp Gly Gln Gly
            100                 105                 110
Thr Met Val Thr Val Ser Ser
            115

<210> 8
<211> 114
<212> PRT
<213> Homo sapiens

<400> 8
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Asn
                20                  25                  30
Ser Asn Asn Lys Lys Tyr Leu Ala Trp Tyr Gln Gln Lys Pro Gly Gln
                35                  40                  45
Pro Pro Lys Leu Leu Ile Tyr Trp Ala Ser Thr Arg Glu Ser Gly Val
        50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Ser Thr Pro Trp Thr Phe Gly Gln Gly Thr Lys Val Glu Ile
            100                 105                 110
Lys Arg

<210>   9
<211>   8
<212>   PRT
<213>   Homo sapiens

<400>   9

Leu Val Trp Val Pro Ser Asp Lys
1               5

<210>   10
<211>   16
<212>   PRT

<213> Homo sapiens

<400> 10

Val Ser His Leu Leu Gly Ile Asn Val Thr Asp Phe Thr Arg Gly Ile
1               5                   10                  15


<210> 11
<211> 12
<212> PRT
<213> Homo sapiens

<400> 11

Glu Gln Ala Asp Phe Ala Ile Glu Ala Leu Ala Lys
1               5                   10


<210> 12
<211> 17
<212> PRT
<213> Homo Sapiens

<400> 12

Asp Val Asp Arg Ile Ile Gly Leu Asp Gln Val Ala Gly Met Ser Glu
1               5                   10                  15


Thr


<210> 13
<211> 15
<212> PRT
<213> Homo Sapiens

<400> 13

Thr Glu Leu Glu Asp Thr Leu Asp Ser Thr Ala Ala Gln Gln Glu
1               5                   10                  15


<210> 14
<211> 12
<212> PRT
<213> Homo Sapiens

<400> 14

Ala Leu Glu Ser Gln Leu Gln Asp Thr Gln Glu Leu
1               5                   10


<210> 15
<211> 16
<212> PRT
<213> Homo Sapiens

<400> 15

Ser Met Glu Ala Glu Met Ile Gln Leu Gln Glu Glu Leu Ala Ala Ala
1               5                   10                  15

<210> 16
<211> 17
<212> PRT
<213> Homo Sapiens

<400> 16

Arg Arg Leu Glu Ala Arg Ile Ala Gln Leu Glu Glu Glu Leu Glu Glu
1               5                   10                  15

Glu


<210> 17
<211> 1960
<212> PRT
<213> Homo Sapiens

<400> 17

Met Ala Gln Gln Ala Ala Asp Lys Tyr Leu Tyr Val Asp Lys Asn Phe
1               5                   10                  15

Ile Asn Asn Pro Leu Ala Gln Ala Asp Trp Ala Ala Lys Lys Leu Val
                20                  25                  30

Trp Val Pro Ser Asp Lys Ser Gly Phe Glu Pro Ala Ser Leu Lys Glu
        35                  40                  45

Glu Val Gly Glu Glu Ala Ile Val Glu Leu Val Glu Asn Gly Lys Lys
        50                  55                  60

Val Lys Val Asn Lys Asp Asp Ile Gln Lys Met Asn Pro Pro Lys Phe
65                  70                  75                  80

Ser Lys Val Glu Asp Met Ala Glu Leu Thr Cys Leu Asn Glu Ala Ser
                85                  90                  95

Val Leu His Asn Leu Lys Glu Arg Tyr Tyr Ser Gly Leu Ile Tyr Thr
                100                 105                 110

Tyr Ser Gly Leu Phe Cys Val Val Ile Asn Pro Tyr Lys Asn Leu Pro
        115                 120                 125

Ile Tyr Ser Glu Glu Ile Val Glu Met Tyr Lys Gly Lys Lys Arg His
        130                 135                 140

Glu Met Pro Pro His Ile Tyr Ala Ile Thr Asp Thr Ala Tyr Arg Ser
145                 150                 155                 160

Met Met Gln Asp Arg Glu Asp Gln Ser Ile Leu Cys Thr Gly Glu Ser
                165                 170                 175

Gly Ala Gly Lys Thr Glu Asn Thr Lys Lys Val Ile Gln Tyr Leu Ala

```
                    180                    185                       190

Tyr Val Ala Ser Ser His Lys Ser Lys Lys Asp Gln Gly Glu Leu Glu
        195              200                 205

Arg Gln Leu Leu Gln Ala Asn Pro Ile Leu Glu Ala Phe Gly Asn Ala
    210              215                 220

Lys Thr Val Lys Asn Asp Asn Ser Ser Arg Phe Gly Lys Phe Ile Arg
225              230                 235                     240

Ile Asn Phe Asp Val Asn Gly Tyr Ile Val Gly Ala Asn Ile Glu Thr
                245                 250                     255

Tyr Leu Leu Glu Lys Ser Arg Ala Ile Arg Gln Ala Lys Glu Glu Arg
            260                 265                 270

Thr Phe His Ile Phe Tyr Tyr Leu Leu Ser Gly Ala Gly Glu His Leu
        275                 280                 285

Lys Thr Asp Leu Leu Leu Glu Pro Tyr Asn Lys Tyr Arg Phe Leu Ser
    290                 295                 300

Asn Gly His Val Thr Ile Pro Gly Gln Gln Asp Lys Asp Met Phe Gln
305                 310                 315                     320

Glu Thr Met Glu Ala Met Arg Ile Met Gly Ile Pro Glu Glu Glu Gln
                325                 330                     335

Met Gly Leu Leu Arg Val Ile Ser Gly Val Leu Gln Leu Gly Asn Ile
            340                 345                 350

Val Phe Lys Lys Glu Arg Asn Thr Asp Gln Ala Ser Met Pro Asp Asn
        355                 360                 365

Thr Ala Ala Gln Lys Val Ser His Leu Leu Gly Ile Asn Val Thr Asp
    370                 375                 380

Phe Thr Arg Gly Ile Leu Thr Pro Arg Ile Lys Val Gly Arg Asp Tyr
385                 390                 395                     400

Val Gln Lys Ala Gln Thr Lys Glu Gln Ala Asp Phe Ala Ile Glu Ala
                405                 410                     415

Leu Ala Lys Ala Thr Tyr Glu Arg Met Phe Arg Trp Leu Val Leu Arg
            420                 425                 430

Ile Asn Lys Ala Leu Asp Lys Thr Lys Arg Gln Gly Ala Ser Phe Ile
        435                 440                 445
```

Gly Ile Leu Asp Ile Ala Gly Phe Glu Ile Phe Asp Leu Asn Ser Phe
450 455 460

Glu Gln Leu Cys Ile Asn Tyr Thr Asn Glu Lys Leu Gln Gln Leu Phe
465 470 475 480

Asn His Thr Met Phe Ile Leu Glu Gln Glu Glu Tyr Gln Arg Glu Gly
485 490 495

Ile Glu Trp Asn Phe Ile Asp Phe Gly Leu Asp Leu Gln Pro Cys Ile
500 505 510

Asp Leu Ile Glu Lys Pro Ala Gly Pro Pro Gly Ile Leu Ala Leu Leu
515 520 525

Asp Glu Glu Cys Trp Phe Pro Lys Ala Thr Asp Lys Ser Phe Val Glu
530 535 540

Lys Val Met Gln Glu Gln Gly Thr His Pro Lys Phe Gln Lys Pro Lys
545 550 555 560

Gln Leu Lys Asp Lys Ala Asp Phe Cys Ile Ile His Tyr Ala Gly Lys
565 570 575

Val Asp Tyr Lys Ala Asp Glu Trp Leu Met Lys Asn Met Asp Pro Leu
580 585 590

Asn Asp Asn Ile Ala Thr Leu Leu His Gln Ser Ser Asp Lys Phe Val
595 600 605

Ser Glu Leu Trp Lys Asp Val Asp Arg Ile Ile Gly Leu Asp Gln Val
610 615 620

Ala Gly Met Ser Glu Thr Ala Leu Pro Gly Ala Phe Lys Thr Arg Lys
625 630 635 640

Gly Met Phe Arg Thr Val Gly Gln Leu Tyr Lys Glu Gln Leu Ala Lys
645 650 655

Leu Met Ala Thr Leu Arg Asn Thr Asn Pro Asn Phe Val Arg Cys Ile
660 665 670

Ile Pro Asn His Glu Lys Lys Ala Gly Lys Leu Asp Pro His Leu Val
675 680 685

Leu Asp Gln Leu Arg Cys Asn Gly Val Leu Glu Gly Ile Arg Ile Cys
690 695 700

Arg Gln Gly Phe Pro Asn Arg Val Val Phe Gln Glu Phe Arg Gln Arg
705 710 715 720

Tyr Glu Ile Leu Thr Pro Asn Ser Ile Pro Lys Gly Phe Met Asp Gly
          725               730              735

Lys Gln Ala Cys Val Leu Met Ile Lys Ala Leu Glu Leu Asp Ser Asn
          740               745              750

Leu Tyr Arg Ile Gly Gln Ser Lys Val Phe Phe Arg Ala Gly Val Leu
          755               760            765

Ala His Leu Glu Glu Glu Arg Asp Leu Lys Ile Thr Asp Val Ile Ile
770               775              780

Gly Phe Gln Ala Cys Cys Arg Gly Tyr Leu Ala Arg Lys Ala Phe Ala
785           790            795           800

Lys Arg Gln Gln Gln Leu Thr Ala Met Lys Val Leu Gln Arg Asn Cys
          805               810            815

Ala Ala Tyr Leu Lys Leu Arg Asn Trp Gln Trp Trp Arg Leu Phe Thr
          820               825            830

Lys Val Lys Pro Leu Leu Gln Val Ser Arg Gln Glu Glu Glu Met Met
          835               840            845

Ala Lys Glu Glu Glu Leu Val Lys Val Arg Glu Lys Gln Leu Ala Ala
          850               855            860

Glu Asn Arg Leu Thr Glu Met Glu Thr Leu Gln Ser Gln Leu Met Ala
865             870            875           880

Glu Lys Leu Gln Leu Gln Glu Gln Leu Gln Ala Glu Thr Glu Leu Cys
          885               890           895

Ala Glu Ala Glu Glu Leu Arg Ala Arg Leu Thr Ala Lys Lys Gln Glu
          900               905           910

Leu Glu Glu Ile Cys His Asp Leu Glu Ala Arg Val Glu Glu Glu Glu
          915               920           925

Glu Arg Cys Gln His Leu Gln Ala Glu Lys Lys Lys Met Gln Gln Asn
          930               935           940

Ile Gln Glu Leu Glu Glu Gln Leu Glu Glu Glu Glu Ser Ala Arg Gln
945             950            955          960

Lys Leu Gln Leu Glu Lys Val Thr Thr Glu Ala Lys Leu Lys Lys Leu
          965               970          975

Glu Glu Glu Gln Ile Ile Leu Glu Asp Gln Asn Cys Lys Leu Ala Lys
          980               985          990

Glu Lys Lys Leu Leu Glu Asp Arg  Ile Ala Glu Phe Thr  Thr Asn Leu
       995                  1000                 1005

Thr Glu  Glu Glu Glu Lys Ser  Lys Ser Leu Ala Lys  Leu Lys Asn
    1010                  1015                  1020

Lys His  Glu Ala Met Ile Thr  Asp Leu Glu Glu Arg  Leu Arg Arg
    1025                  1030                  1035

Glu Glu  Lys Gln Arg Gln Glu  Leu Glu Lys Thr Arg  Arg Lys Leu
    1040                  1045                  1050

Glu Gly  Asp Ser Thr Asp Leu  Ser Asp Gln Ile Ala  Glu Leu Gln
    1055                  1060                  1065

Ala Gln  Ile Ala Glu Leu Lys  Met Gln Leu Ala Lys  Lys Glu Glu
    1070                  1075                  1080

Glu Leu  Gln Ala Ala Leu Ala  Arg Val Glu Glu Glu  Ala Ala Gln
    1085                  1090                  1095

Lys Asn  Met Ala Leu Lys Lys  Ile Arg Glu Leu Glu  Ser Gln Ile
    1100                  1105                  1110

Ser Glu  Leu Gln Glu Asp Leu  Glu Ser Glu Arg Ala  Ser Arg Asn
    1115                  1120                  1125

Lys Ala  Glu Lys Gln Lys Arg  Asp Leu Gly Glu Glu  Leu Glu Ala
    1130                  1135                  1140

Leu Lys  Thr Glu Leu Glu Asp  Thr Leu Asp Ser Thr  Ala Ala Gln
    1145                  1150                  1155

Gln Glu  Leu Arg Ser Lys Arg  Glu Gln Glu Val Asn  Ile Leu Lys
    1160                  1165                  1170

Lys Thr  Leu Glu Glu Glu Ala  Lys Thr His Glu Ala  Gln Ile Gln
    1175                  1180                  1185

Glu Met  Arg Gln Lys His Ser  Gln Ala Val Glu Glu  Leu Ala Glu
    1190                  1195                  1200

Gln Leu  Glu Gln Thr Lys Arg  Val Lys Ala Asn Leu  Glu Lys Ala
    1205                  1210                  1215

Lys Gln  Thr Leu Glu Asn Glu  Arg Gly Glu Leu Ala  Asn Glu Val
    1220                  1225                  1230

Lys Val  Leu Leu Gln Gly Lys  Gly Asp Ser Glu His  Lys Arg Lys

20

1235            1240            1245

Lys Val Glu Ala Gln Leu Gln Glu Leu Gln Val Lys Phe Asn Glu
     1250            1255            1260

Gly Glu Arg Val Arg Thr Glu Leu Ala Asp Lys Val Thr Lys Leu
     1265            1270            1275

Gln Val Glu Leu Asp Asn Val Thr Gly Leu Leu Ser Gln Ser Asp
     1280            1285            1290

Ser Lys Ser Ser Lys Leu Thr Lys Asp Phe Ser Ala Leu Glu Ser
     1295            1300            1305

Gln Leu Gln Asp Thr Gln Glu Leu Leu Gln Glu Glu Asn Arg Gln
     1310            1315            1320

Lys Leu Ser Leu Ser Thr Lys Leu Lys Gln Val Glu Asp Glu Lys
     1325            1330            1335

Asn Ser Phe Arg Glu Gln Leu Glu Glu Glu Glu Glu Ala Lys His
     1340            1345            1350

Asn Leu Glu Lys Gln Ile Ala Thr Leu His Ala Gln Val Ala Asp
     1355            1360            1365

Met Lys Lys Lys Met Glu Asp Ser Val Gly Cys Leu Glu Thr Ala
     1370            1375            1380

Glu Glu Val Lys Arg Lys Leu Gln Lys Asp Leu Glu Gly Leu Ser
     1385            1390            1395

Gln Arg His Glu Glu Lys Val Ala Ala Tyr Asp Lys Leu Glu Lys
     1400            1405            1410

Thr Lys Thr Arg Leu Gln Gln Glu Leu Asp Asp Leu Leu Val Asp
     1415            1420            1425

Leu Asp His Gln Arg Gln Ser Ala Cys Asn Leu Glu Lys Lys Gln
     1430            1435            1440

Lys Lys Phe Asp Gln Leu Leu Ala Glu Glu Lys Thr Ile Ser Ala
     1445            1450            1455

Lys Tyr Ala Glu Glu Arg Asp Arg Ala Glu Ala Glu Ala Arg Glu
     1460            1465            1470

Lys Glu Thr Lys Ala Leu Ser Leu Ala Arg Ala Leu Glu Glu Ala
     1475            1480            1485

```
Met Glu  Gln Lys Ala Glu Leu  Glu Arg Leu Asn Lys  Gln Phe Arg
    1490                  1495              1500

Thr Glu  Met Glu Asp Leu Met  Ser Ser Lys Asp Asp  Val Gly Lys
    1505                  1510              1515

Ser Val  His Glu Leu Glu Lys  Ser Lys Arg Ala Leu  Glu Gln Gln
    1520                  1525              1530

Val Glu  Glu Met Lys Thr Gln  Leu Glu Glu Leu Glu  Asp Glu Leu
    1535                  1540              1545

Gln Ala  Thr Glu Asp Ala Lys  Leu Arg Leu Glu Val  Asn Leu Gln
    1550                  1555              1560

Ala Met  Lys Ala Gln Phe Glu  Arg Asp Leu Gln Gly  Arg Asp Glu
    1565                  1570              1575

Gln Ser  Glu Glu Lys Lys Lys  Gln Leu Val Arg Gln  Val Arg Glu
    1580                  1585              1590

Met Glu  Ala Glu Leu Glu Asp  Glu Arg Lys Gln Arg  Ser Met Ala
    1595                  1600              1605

Val Ala  Ala Arg Lys Lys Leu  Glu Met Asp Leu Lys  Asp Leu Glu
    1610                  1615              1620

Ala His  Ile Asp Ser Ala Asn  Lys Asn Arg Asp Glu  Ala Ile Lys
    1625                  1630              1635

Gln Leu  Arg Lys Leu Gln Ala  Gln Met Lys Asp Cys  Met Arg Glu
    1640                  1645              1650

Leu Asp  Asp Thr Arg Ala Ser  Arg Glu Glu Ile Leu  Ala Gln Ala
    1655                  1660              1665

Lys Glu  Asn Glu Lys Lys Leu  Lys Ser Met Glu Ala  Glu Met Ile
    1670                  1675              1680

Gln Leu  Gln Glu Glu Leu Ala  Ala Ala Glu Arg Ala  Lys Arg Gln
    1685                  1690              1695

Ala Gln  Gln Glu Arg Asp Glu  Leu Ala Asp Glu Ile  Ala Asn Ser
    1700                  1705              1710

Ser Gly  Lys Gly Ala Leu Ala  Leu Glu Glu Lys Arg  Arg Leu Glu
    1715                  1720              1725

Ala Arg  Ile Ala Gln Leu Glu  Glu Glu Leu Glu Glu  Glu Gln Gly
    1730                  1735              1740
```

22

```
Asn Thr  Glu Leu Ile Asn Asp  Arg Leu Lys Lys Ala  Asn Leu Gln
    1745                1750                1755

Ile Asp  Gln Ile Asn Thr Asp  Leu Asn Leu Glu Arg  Ser His Ala
    1760                1765                1770

Gln Lys  Asn Glu Asn Ala Arg  Gln Gln Leu Glu Arg  Gln Asn Lys
    1775                1780                1785

Glu Leu  Lys Val Lys Leu Gln  Glu Met Glu Gly Thr  Val Lys Ser
    1790                1795                1800

Lys Tyr  Lys Ala Ser Ile Thr  Ala Leu Glu Ala Lys  Ile Ala Gln
    1805                1810                1815

Leu Glu  Glu Gln Leu Asp Asn  Glu Thr Lys Glu Arg  Gln Ala Ala
    1820   .             1825                1830

Cys Lys  Gln Val Arg Arg Thr  Glu Lys Lys Leu Lys  Asp Val Leu
    1835                1840                1845

Leu Gln  Val Asp Asp Glu Arg  Arg Asn Ala Glu Gln  Tyr Lys Asp
    1850                1855                1860

Gln Ala  Asp Lys Ala Ser Thr  Arg Leu Lys Gln Leu  Lys Arg Gln
    1865                1870                1875

Leu Glu  Glu Ala Glu Glu Glu  Ala Gln Arg Ala Asn  Ala Ser Arg
    1880                1885                1890

Arg Lys  Leu Gln Arg Glu Leu  Glu Asp Ala Thr Glu  Thr Ala Asp
    1895                1900                1905

Ala Met  Asn Arg Glu Val Ser  Ser Leu Lys Asn Lys  Leu Arg Arg
    1910                1915                1920

Gly Asp  Leu Pro Phe Val Val  Pro Arg Arg Met Ala  Arg Lys Gly
    1925                1930                1935

Ala Gly  Asp Gly Ser Asp Glu  Glu Val Asp Gly Lys  Ala Asp Gly
    1940                1945                1950

Ala Glu  Ala Lys Pro Ala Glu
    1955                1960
```

```
<210>  18
<211>  26
<212>  DNA
<213>  Homo Sapiens

<400>  18
```

```
cgggtaccat ggcacagcaa gctgcc                                    26

<210>  19
<211>  26
<212>  DNA
<213>  Homo Sapiens

<400>  19
gactagtctt ctcgtcctcc acctgc                                    26

<210>  20
<211>  10
<212>  PRT
<213>  Homo sapiens

<400>  20
Ala Leu Pro Gly Ala Phe Lys Thr Arg Lys
                5                      10
<210>  21
<211>  10
<212>  PRT
<213>  Homo sapiens

<400>  21

Glu Glu Leu Val Lys Val Arg Glu Lys Gln
                5                      10
<210>  22
<211>  10
<212>  PRT
<213>  Homo sapiens

<400>  22
Ala Asp Gly Ala Glu Ala Lys Pro Ala Glu
                5                      10
```

**Description**

Technical Field

The present invention relates to an antigen having a part which is exposed on the surface of a cell at the formation of a tumor mass. More specifically, the present invention relates to a useful medicament which recognizes, as an antigen, a part of a non-muscular myosin heavy chain type A or a mutant thereof which is exposed to the cell surface in a solid tumor.

Background of the Invention

Currently, studies on cancer targeting agents using antibodies for cancer cells are in progress to obtain high efficacy and safety as antitumor agents. For example, a human monoclonal antibody screened by its reactivity with gastric cancer and colon cancer is known as GAH antibody (cf. EP-A-526700 and EP-A-520499), and development of a drug-containing liposome on which the antibody was bound (cf. EP-A-526700) is in progress.

On the other hand, it is known that the variation of antibodies which recognize the cells depends on the kinds of cells which proceed to malignancies. When a drug-containing liposome bound to the antibody is used as a cancer targeting agent, it is considered that identification of an antigen which is recognized by the antibody is necessary for obtaining higher efficacy and safety as an antitumor agent. However, regarding the GAH antibody, an antigen recognized by the antibody has not so far been identified.

Also, although there are reports stating that an antibody obtained by immunizing a rabbit with a myosin heavy chain purified from a mouse fibroblast cell line L929 reacts with surfaces of L929 and other cells [cf. Willingham M.C., *Proc. Natl. Acad. Sci. U.S.A.*, 71, 4144 (1974), and Olden K., *Cell*, 8, 383-390 (1976)], there are no reports on the exposure of a human non-muscular myosin heavy chain type A (hereinafter referred sometimes to as "nmMHCA") to the cell surface by carcinogenesis, and there are no reports in which the protein is a cancer-associated antigen.

Patent Reference 1: EP-A-526700
Patent Reference 2: EP-A-520499
Non-patent Reference 1: Willingham M.C., *Proc. Natl. Acad. Sci. U.S.A.*, 71, 4144 (1974)
Non-patent Reference 2: Olden K., *Cell*, 8, 383-390 (1976)

Disclosure of the Invention

In order to solve the above problems, the present inventors have conducted intensive studies and found as a result that an antibody represented by the human monoclonal antibody disclosed in EP-A-520499 (GAH antibody) recognizes an antigen having a part which is exposed on the surface of a cell at the formation of a tumor mass in the cell.

That is, the present invention relates to the followings.

(1) An antigen having a part which is exposed on the surface of a cell at the formation of a tumor mass.
(2) The above antigen, wherein the tumor mass is a solid tumor formed by subcutaneous transplantation of a cultured cancer cell.
(3) The above antigen, wherein the existing amount of the antigen of the solid tumor is increased in comparison with that of a cultured cell of the solid tumor.
(4) The above antigen, wherein the existing amount of the antigen of the solid tumor on the cell surface is increased in comparison with that of a cultured cell of the solid tumor.
(5) The above antigen, which is a cytoskeleton protein or a mutant thereof.
(6) The above antigen, which is myosin or a mutant thereof.
(7) The above antigen, which is a non-muscular myosin heavy chain type A or a mutant thereof.
(8) The above antigen, which is a part of a non-muscular myosin heavy chain type A or a mutant thereof.
(9) The above antigen, which is a sequence of a C-terminal domain of the protein sequence of a non-muscular myosin heavy chain type A or a mutant thereof.
(10) The above antigen, wherein the sequence of a C-terminal domain of the protein sequence is a sequence of the residue at position 600 to the residue at position 1,960 from the N-terminal of SEQ ID NO:17 in the Sequence Listing.
(11) The above antigen, wherein the sequence of a C-terminal domain of the protein sequence is any one of SEQ ID NOs:20, 21 and 22.
(12) A ligand which recognizes the above antigen.
(13) The above ligand, which is an antibody.

(14) The above ligand, which is a monoclonal antibody.

(15) The above ligand, wherein the monoclonal antibody is a human monoclonal antibody.

(16) The above ligand, which is a cancer reactive monoclonal antibody.

(17) The above ligand, wherein the cancer is gastric cancer, breast cancer, colon cancer or esophageal cancer.

(18) The above ligand, wherein the heavy chain hypervariable region comprises the amino acid sequences of SEQ ID NOs:1, 2 and 3 in the Sequence Listing, and the light chain hypervariable region comprises the amino acid sequences of SEQ ID NOs:4, 5 and 6 in the Sequence Listing.

(19) The above ligand, which comprises a heavy chain variable region comprising the amino acid sequence of SEQ ID NO:7 in the Sequence Listing and a light chain variable region containing the amino acid sequence of SEQ ID NO:8 in the Sequence Listing.

(20) A pharmaceutical composition, which comprises the above ligand.

(21) The above pharmaceutical composition, which is a targeting therapy agent.

(22) The above pharmaceutical composition, which targets at a cancer tissue or a cancer cell.

(23) The above pharmaceutical composition, which comprises an antitumor agent, an antitumor protein, an enzyme, a gene or an isotope for treatment.

(24) The above pharmaceutical composition, which is an antitumor agent.

(25) The above pharmaceutical composition, wherein the cancer is gastric cancer, breast cancer, colon cancer or esophageal cancer.

(26) The above pharmaceutical composition, which comprises liposome.

(27) A labeling agent, which comprises the above ligand.

(28) The above labeling agent, which specifically labels a cancer tissue or a cancer cell.

(29) The above labeling agent, wherein the cancer is gastric cancer, breast cancer, colon cancer or esophageal cancer.

(30) The above labeling agent, which comprises a fluorescent, an enzyme, an isotope or an MRI contrast medium.

(31) A method for treating a cancer disease of a cancer disease patient which expresses the above antigen, which comprises administering the above pharmaceutical composition.

(32) A method for treating a cancer disease of a cancer disease patient having a cell which can be labeled by the above labeling agent, which comprises administering the above pharmaceutical composition.

(33) The above ligand, wherein the binding activity of the ligand which recognizes the above antigen to the antigen is from $0.5 \times 10^6$ units/mg to $2.0 \times 10^6$ units/mg.

(34) The above ligand, wherein the binding activity is from $0.7 \times 10^6$ units/mg to $1.5 \times 10^6$ units/mg, from $0.7 \times 10^6$ units/mg to $1.3 \times 10^6$ units/mg, or from $0.8 \times 10^6$ units/mg to $1.2 \times 10^6$ units/mg.

(35) The above ligand, wherein the binding activity is from $0.8 \times 10^6$ units/mg to $1.2 \times 10^6$ units/mg.

Brief Description of the Drawings

Fig. 1 shows reactivity of GAH antibody with cultured cells and transplantation-derived cells of MKN45.

Fig. 2 is a photograph showing results of the evaluation of reactivity of GAH and anti-nmMHCA antibodies in tissue sections of stably expressing nmMHCA.

Fig. 3 shows GAH antibody binding numbers, as a result of the evaluation of cell surface reactivity of GAH using a cell line stably expressing nmMHCA.

Fig. 4 shows anti-nmMHCA antibody binding numbers, as results of the evaluation of cell surface reactivity of anti-nmMHCA antibody using a cell line stably expressing nmMHCA.

Fig. 5 shows results of the evaluation of reactivity of anti-nmMHCA peptide antibody to the surface of MKN45 cultured cells and tumor cells formed by subcutaneous transplantation of MKN45.

Fig. 6 shows a relationship between the tumor growth inhibitory effect and the antigen amount per cell.

Best Mode for Carrying out the Invention

The present invention is described below in detail.

The cell of the present invention includes cells derived from stomach, large intestine, esophagus, mammary gland, lungs, pancreas, liver, kidney, ovary or uterus. Preferred examples include cells derived from stomach, large intestine, esophagus or mammary gland. More preferred examples include cells derived from large intestine.

The tumor mass of the present invention may be any tumor mass, so long as it forms an aggregate of tumor cells visibly or microscopically. Preferred examples include those in which a normal tissue is spontaneously transformed to solid tumor by tumorigenesis and those in which a cancer cell is proliferated by the transplantation of the cell. More preferred is a solid tumor formed by subcutaneous transplantation of a cultured cancer cell.

The term exposure as used herein means that an internal substance appears on the surface of cell membrane or that the internal substance is exposed by the peeling of a substance covering the surface, and preferably it means that a part of an antigen appears on the surface of a cell. More preferably, it means that the C-terminal domain of the protein sequence of an antigen appears on the surface of a cell.

Examples of the antigen of the present invention include a protein, a glycoprotein, a protein-lipid complex and mutants thereof, which are considered to function as a cytoskeleton and an organelle in normal cells. Preferred is one which functions as an antigen when a cell forms a tumor mass. More preferred are myosin, actin, tropomyosin, vimentin, cytokeratin and the like or mutants thereof. Most preferred is human non-muscular myosin heavy chain type A (nmMHCA). Herein, nmMHCA is obtained by preparing a gene in accordance with the method of Toothaker L.E. *et al.* [*Blood*, 78(7), 1826-1833 (1991)] or the method of Saez C.G. *et al.* [*Proc. Natl. Acad. Sci. USA,* 87(3), 1164-8 (1990, Feb)], and expressing the protein using the gene in accordance with *Molecular Cloning, A Laboratory Manual* [Second Edition, Cold Spring Harbor Laboratory Press (1989)].

Examples of the sequence of a C-terminal domain of the protein of the present invention include a sequence of the residue at position 600 to the residue at position 1,960 from the N-terminal side of nmMHCA represented by SEQ ID NO:17 in the Sequence Listing, and preferred examples include the sequence of SEQ ID NO:20, 21 or 22 in the Sequence Listing.

Examples of comparison of the solid tumor of the present invention with a cultured cell of the solid tumor include a method in which cultured cancer cells are compared with solid tumor-derived cancer cells separated from a solid tumor formed by once transplanting the cultured cells to an animal, a method in which cancer cells separated from solid cancer tissues of a patient are compared with cultured cancer cells adapted by once culturing the cancer cells *in vitro*, and the like, although not limited thereto.

The existing amount on the cell surface of the present invention means the existing amount of an antigen in the entire cell or the existing amount of the antigen only on the cell surface, and preferably means the existing amount of the antigen only on the cell surface. The existing amount on the cell surface can be determined, for example, by flow cytometry, although not limited thereto.

Examples of the increase of the present invention include increase by 3 times or more, preferred examples include increase by 4 times or more, and more preferred examples include increase by 10 times or more.

Examples of the mutant of the present invention include those which have amino acid sequences in which one or several amino acids are deleted, substituted or added, or those in which three-dimensional structure of the normal protein is modified.

Examples of the ligand of the present invention include proteins, for example, various antibodies, growth factors or proliferation factors such as fibroblast growth factor (FGF) and epidermal growth factor (EGF), and preferred examples include antibodies. Also, examples of the antibodies include polyclonal antibodies of various animals, mouse monoclonal antibodies, human-mouse chimeric antibodies or human type monoclonal antibodies and human monoclonal antibodies, and preferred examples include human monoclonal antibodies. More preferred examples include cancer reactive human monoclonal antibodies, and a human monoclonal antibody disclosed in EP-A-520499 (GAH antibody).

In the GAH antibody, the amino acid sequences of SEQ ID NOs:1, 2 and 3 in the Sequence Listing are called hypervariable region among heavy chain variable regions, and the amino acid sequences of SEQ ID NOs:4, 5 and 6 in the Sequence Listing are called hypervariable region among light chain variable regions. The regions determine specificity of immunoglobulin as an antibody and binding affinity of an antigenic determinant with the antibody, and are also called complementarity determining regions. Accordingly, regions other than the hypervariable regions may be derived from other antibodies. That is, it is considered that an antibody having hypervariable regions similar to those of the GAH antibody can be used in the present invention in the same manner as the GAH antibody.

Thus, in a preferred monoclonal antibody to be used in the present invention, the amino acid sequences of SEQ ID NOs:1, 2 and 3 in the Sequence Listing are contained in the heavy chain hypervariable region, and the amino acid sequences of SEQ ID NOs:4, 5 and 6 in the Sequence Listing are contained in the light chain hypervariable region. In these amino acid sequences, generally, SEQ ID NOs:1, 2 and 3 in the Sequence Listing and SEQ ID NOs:4, 5 and 6 in the Sequence Listing are contained in this order from the N-terminal region, in the three hypervariable regions of each chain of the heavy chain and light chain. According to the present invention, those in which modifications such as substitution, insertion, deletion or addition of some amino acids were carried out within a range of not spoiling their reactivity with cancers are also included in the monoclonal antibodies which can be used in the present invention.

The monoclonal antibody of the present invention can be obtained by preparing a hybridoma of a patient-derived lymphocyte with a mouse myeloma cell, and selecting one having the above specified amino acid sequence.

The hybridoma is obtained in accordance with the method of A. Imam *et al.* [*Cancer Research*, 45, 263 (1985)], by firstly isolating lymphocyte from a lymph node belonging to a cancer excised from a cancer patient, and fusing it with a mouse myeloma cell using polyethylene glycol. Using supernatants of the thus obtained hybridomas, hybridomas capable of producing antibodies which are positive by enzyme immunoassay for various cancer cell lines fixed with p-formaldehyde are selected and their cloning is carried out.

Subsequently, monoclonal antibodies are purified from supernatants of the hybridomas by a conventional method [R.C. Duhamel *et al., J. Immunol. Methods*, 31, 211 (1979)] and labeled with a fluorescent material to check their reactivity with intact cancer cell lines, various erythrocytes, leukocytes and so on by flow cytometry. Antibodies which show reactivity with intact cancer cell lines and antibodies which do not show reactivity with erythrocytes and leukocytes are selected. In addition, by comparing the reactivity with cancer cells isolated from a cancer tissue excised from a cancer patient and normal cells isolated from a non-cancer part of the same tissue of the same patient, an antibody in which larger numbers of its molecules are bound to the cancer cells and which does not react with normal cells or has a similar degree of reactivity with an antibody derived from a healthy person.

The nucleotide sequence of DNA encoding the antibody produced by the thus selected hybridoma is obtained, for example, by the following method. From the antibody producing hybridoma, mRNA is prepared by the guanidine thiocyanate-lithium chloride method [Casara *et al., DNA,* 2, 329 (1983)], and its cDNA library is prepared using oligo (dT) primer. Next, (dG) tailing of cDNA is carried out, and cDNA encoding the antibody is amplified by PCR using a partial sequence as the probe, which is common to poly C which hybridizes with this dG tail and the human antibody heavy chain gene and light chain gene already obtained. Thereafter, the termini of the DNA are smooth-ended, the DNA extracted from a gel by electrophoresis is inserted into a cloning vector such as pUC119, and its nucleotide sequence is determined by the dideoxy method of Sanger *et al.* [*Proc. Natl. Acad. Sci. U.S.A.*, 74, 5463 (1977)]. Based on this nucleotide sequence, an antibody having the above specified amino acid sequence can be selected.

In addition, the monoclonal antibody to be used in the present invention can also be prepared by genetic engineering techniques.

The particularly preferable monoclonal antibody of the present invention is one in which the heavy chain variable region and the light chain variable region are represented by the amino acid sequences of SEQ ID NOs:7 and 8, respectively, in the Sequence Listing. The nucleotide sequences for the constant regions of the heavy chain and the light chain may be those which have the same sequences described, for example, in *Nucleic Acids Research,* 14, 1779 (1986), *The Journal of Biological Chemistry*, 257, 1516 (1982) and *Cell,* 22, 197 (1980).

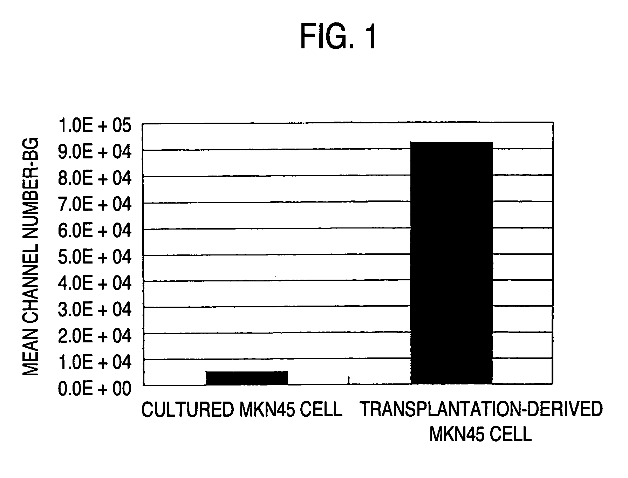

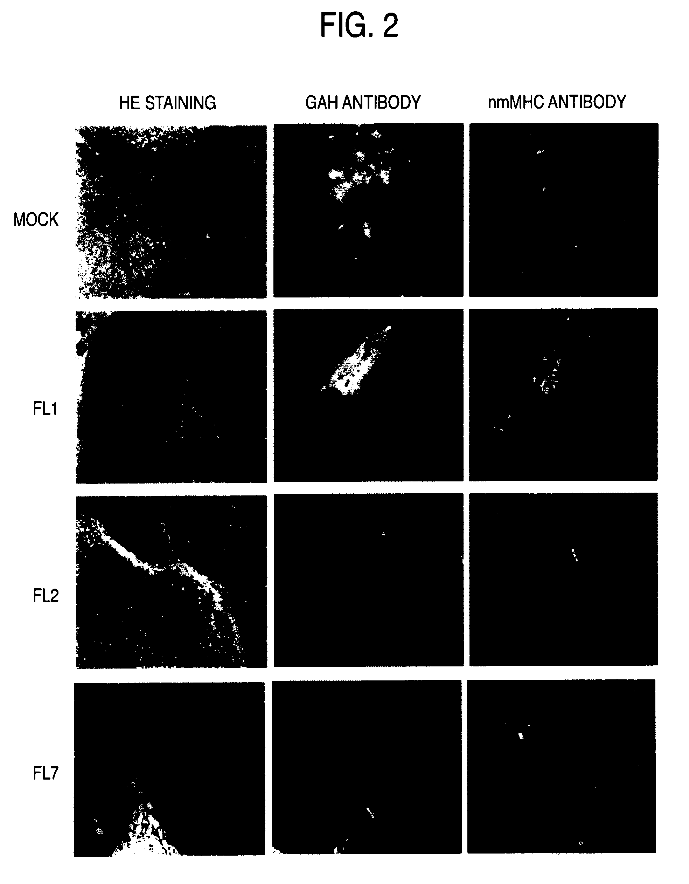

This antibody can be obtained by culturing a hybridoma capable of producing this antibody using fetal bovine serum-containing eRDF, RPMI 1640 culture medium or the like, or by chemically synthesizing a gene in which the DNA molecules encoding variable regions including the above specified hypervariable regions are further ligated with each of DNA molecules encoding constant regions of the heavy chain and the light chain, inserting it into various conventionally known expression vectors capable of expressing the gene, for example, pKCR(ΔE)/H and pKCRD as expression vectors for animal cells, that can be constructed from pKCRH2 [Mishina *et al., Nature*, 307, 605 (1984)] by the procedure shown in Fig. 1 or Fig. 2 of EP-A-520499, and then expressing it in a host such as CHO cell (Chinese hamster ovary cell). For example, a *Hin*dIII site is added to both termini of the heavy chain gene and the product is inserted into the *Hin*dIII site of pKCR(ΔE)/H, and a selectable marker gene such as DHFR gene is inserted into *Sal*I site of this plasmid. On the other hand, an *Eco*RI site is added to both termini of the light chain gene and the product is inserted into *Eco*RI of pKCRD, and the DHFR gene is also inserted into *Sal*I site of this plasmid. By introducing both plasmids into a cell such as CHO dhfr⁻ [Urlaub G. & Chasin L.A., *Proc. Natl. Acad. Sci. U.S.A.*, 77, 4216 (1980)] by the calcium phosphate method and culturing the cell using nucleotide-free αMEM culture medium or the like, a cell capable of producing the antibody can be further obtained by selecting from the cells which can grow in the medium. The antibody is purified from a culture medium obtained by culturing these cells by adsorbing it to a column or the like in which protein A is linked to a carrier such as Cellulofine or agarose, and then eluting it.

Examples of the cancer of the present invention include cancers having a possibility in that the antibody has a reactivity therewith when, for example, the antibody is used as a single chain antibody (scFv), a whole antibody or a fragment thereof. Preferred examples include gastric cancer, colon cancer, esophageal cancer, lung cancer, breast cancer, hepatic cancer, ovarian cancer, uterine cancer and pancreatic cancer. More preferred examples include gastric cancer, breast cancer, colon cancer and esophageal cancer.

Examples of the pharmaceutical composition of the present invention include a ligand alone and a targeting therapy agent in which an active substance is linked to the ligand, and preferred examples include the targeting therapy agent. Examples of the targeting therapy agent include those in which an active substance is directly linked to a ligand, an active substance is linked to the ligand via a water-soluble polymer or an active substance-containing fine particle is linked to the ligand. Examples of the fine particle include microsphere, micelle and liposome, and preferred examples include liposome. A pharmaceutical preparation and a labeling agent may be contained in the liposome. Examples of the pharmaceutical preparation to be contained therein include antitumor agents such as adriamycin, daunomycin, vinblastine, cisplatin, mitomycin, bleomycin, actinomycin and fluorouracil (5-FU), pharmaceutically acceptable salts and derivatives thereof. Further examples include a toxic proteins such as ricin A and diphtheria toxin and DNAs encoding them, DNA encoding the cytokine gene ofFNF or its antisense DNA, and nucleotides. Particularly preferred examples include adriamycin. Also, examples of the labeling agent to be contained therein include an imaging agent of radioactive element such as indium or technetium, enzymes such as horseradish peroxidase and alkaline phosphatase, MRI contrast medium of gadolinium, X-ray contrast medium of iodine, ultrasonic contrast medium of $CO_2$, europium derivatives, fluorescent of carboxyfluorescein or illuminant of an N-methylacridium derivative. Examples of the water-soluble polymer derivative include a synthetic polymer of polyethylene glycol, polyacrylamide, polyvinyl pyrrolidone, polyglycerol, polylactic acid, polyglycolic acid or polyamino acid, and preferred examples include polyethylene glycol. It is preferred that the targeting therapy agent targets at a cancer tissue or a cancer cell. Examples of the cancer in this case include gastric cancer, colon cancer, esophageal cancer, lung cancer, breast cancer, hepatic cancer, ovarian cancer, uterine cancer and pancreatic cancer. More preferred examples include gastric cancer, breast cancer, colon cancer and esophageal cancer.

According to the present invention, the activity of an antigen-recognizing ligand to bind to the antigen is measured for, for example, ensuring constancy of the quality of a ligand when the ligand capable of recognizing the antigen is industrially used, though not limited to this purpose. In addition, as the measuring method, for example, there is a method in which a calibration curve is prepared from standard solutions containing predetermined amounts of the antibody using a microplate reader or the like, and the antigen-binding activity of the antibody contained in the test solution is measured, although not limited to this measuring method.

When the binding activity is expressed by a titer, a range of from $0.5 \times 10^6$ to $2.0 \times 10^6$ units/mg can be exemplified, and it is preferably within the range of from $0.7 \times 10^6$ to $1.5 \times 10^6$ units/mg, from $0.7 \times 10^6$ to $1.3 \times 10^6$ units/mg or from $0.8 \times 10^6$ to $1.2 \times 10^6$ units/mg. It is more preferably from $0.8 \times 10^6$ to $1.2 \times 10^6$ units/mg.

The complex of a ligand-linked active substance and a water-soluble polymer derivative can be made into a pharmaceutical preparation, for example, by the method described in EP-A-526700 or EP-A-520499, and the complex can be administered to patients by intravascular administration, bladder administration, intraperitoneal administration, topical administration or the like for the treatment of various diseases such as a cancer. The dose can be optionally selected according to the kind of the antitumor substance as the active ingredient, and when liposome containing doxorubicin is administered, for example, it can be used in 50 mg/kg or less, preferably 10 mg/kg or less, and more preferably 5 mg/kg or less, as the amount of the active ingredient.

Examples

The present invention is described below in more detail with reference to examples, but the present invention is not restricted by the following examples without overstepping its gist.

Example 1

Reactivity comparison of antibodies and detection of surface antigen by immunoprecipitation:

Preparation of tumor cell by subcutaneous transplantation of MKN45

A human gastric cancer cell MKN45 (Immuno-Biological Laboratories) was cultured in a liquid medium eRDF (Gibco) containing 10% fetal bovine serum (Sigma), and the resulting cells were recovered and subcutaneously transplanted under the back of an about 5-week-old BALB/C node mouse (CLEA Japan). The thus formed subcutaneous tumor tissue was extracted, and a cell was isolated from the tissue in accordance with the method of Tokita *et a*l., [*Gan no Rinsho* (*Clinical Cancer*), 32, 1803 (1986)]. The tissue was put on a Teflon sheet spread on a rubber plate, cut into thin pieces by tapping with a razor, and then passed through a nylon mesh (a cell strainer, FALCON) to remove the connective tissue. Cell suspension as the filtrate was centrifuged at 1,500 rpm for 5 minutes (Tomy table centrifuge LC06-SP), and the floated fat and suspended necrosis moiety were discarded and the precipitate was repeatedly washed.

Reactivity comparison of GAH antibody by flow cytometry

Cultured cells of MKN45 or cells of a solid tumor (hereinafter referred to as "tumor cells") formed by subcutaneous transplantation ofMKN45 were allowed to react with the GAH antibody described in EP-A-520499 and EP-A-1174126, which had been labeled with fluorescein isothiocyanate (FITC: Sigma), in a concentration of 20 µg/ml at 4°C for 1 hour, and then washed once with phosphate buffered saline (PBS). Analysis was carried out in PBS containing propidium iodide (PI: Sigma) using a flow cytometer (FACScan: Becton Dickinson). PI-positive cells, namely dead cells, were excluded from the subject of analysis by gating operation. By calculating the mean channel number of FL1 as an index of FITC fluorescence intensity, comparison of the cultured cells and tumor cells of MKN45 was carried out.

The results are shown in Fig. 1. The ordinate shows a value obtained by subtracting a value in the case of not containing the antibody as a background value (BG) from the mean channel number. E is the power of 10.

The GAH antibody showed a higher reactivity of about 18 times for the tumor cells than the cultured cells of MKN45.

Based on this result, it was found that the GAH antibody shows a higher reactivity for the tumor cells in comparison with the cultured cells.

Biotin labeling of the surface of cells and preparation of solubilized supernatant

To cultured cells or tumor cells of MKN45, 1 mg/ml PBS solution of a biotin reagent (sulfoNHS-biotin: PIERCE) was added, followed by incubation at 4°C for 30 minutes on a shaker. Thereafter, the cells were washed with PBS containing 5 mM glycine (Nacalai Tesque) and then with PBS. Pellet of the cells was mixed with a solution of 1% NP 40, aprotinin (Sigma) and nafamostat mesylate (Torii Yakuhin) in 20 mM Tris (Sigma) hydrochloride buffer, pH 7.5, containing 150 mM NaCl and 1 mM EDTA (TNE buffer), stirred, subjected to ultrasonic treatment, allowed to stand on ice for 1 hour and then centrifuged (Tomy table cooling centrifuge MRX-150), and the supernatant was used as a solubilized supernatant of cells.

Immunoprecipitation and detection of biotin-labeled band

A solution of GAH antibody or a healthy person-derived human immunoglobulin (human Igs) (purified from human serum obtained from Scanty Bodies Laboratory using a protein A column (Repligen)) was added to protein A Sepharose CL4B (Pharmacia) which had been equilibrated with PBS, and the thus antibody-linked resin was washed with PBS, and the solubilized supernatant of cells was added thereto, followed by incubation at 4°C overnight on a shaker. After discarding the supernatant by centrifugation, the resin was washed 3 times with TNE buffer containing 0.1% NP 40 (Nacalai Tesque) and extracted with a sample buffer for SDS polyacrylamide gel electrophoresis (SDS-PAGE), and the extract was subjected to SDS-PAGE (4 to 12% gradient gel) and then to Western blotting on PVDF membrane (Millipore). The protein-transferred membrane was incubated at room temperature for 1 hour in PBS containing 0.1% gelatin (Nacalai Tesque) and 0.05% Tween 20 (Nacalai Tesque), and then, in order to detect the biotin-labeled protein, allowed to react with Vectastain ElliteABC (Vector) at room temperature for 1 hour. Konica Immunostain HRP1000 (Konica) was used for the color development. The sample used in lane 1 is an immunoprecipitation product by the GAH antibody of the tumor cell, the sample used in lane 2 is an immunoprecipitation product by the GAH antibody of cultured cell, the sample used in lane 3 is an immunoprecipitation product by the human Igs of tumor cell, and the sample used in lane 4 is an immunoprecipitation product by the human Igs of cultured cell.

On the immunoprecipitation product by the GAH antibody of the tumor cell of lane 1, a GAH antibody-specific band was detected at a position of about 200 kd in molecular weight. A band was hardly detected at the corresponding position on the immunoprecipitation product by the GAH antibody of cultured cell of lane 2.

It was found from this result that the GAH antibody has a reactivity with a protein of about 200 kd. In addition, it was shown that this protein of about 200 kd is exposed on the tumor cell surface.

Amino acid sequence analysis of 200 kd protein

The 200 kd protein specifically detected by immunoprecipitation was cut out from the polyacrylamide gel, treated with lysyl endopeptidase (Wako Pure Chemical Industries) and subjected to reverse phase chromatography, and then amino acid sequence analysis was carried out on the thus obtained peaks (SEQ ID NOs:9 to 16 in the Sequence Listing).

When homology retrieval was carried out based on these sequences, the human non-muscular myosin A chain (nmMHCA) coincided with this 200 kd protein (SEQ ID NO:17 in the Sequence Listing).

From this result, it was found that the protein of 200 kd is nmMHCA.

Detection by anti-non-muscular myosin heavy chain (nmMHC) antibody

The above immunoprecipitation samples were subjected to SDS-PAGE and Western blotting, incubated at room temperature for 1 hour in PBS containing 0.1% gelatin and 0.05% Tween 20, and then allowed to react at room temperature for 1 hour in a solution of anti-nmMHC rabbit polyclonal antibody (Biomedical Technologies). Normal rabbit immunoglobulin (rabbit IgG: Biogenensis) was used as the negative control of the antibody. After the reaction in a solution of a peroxidase-labeled anti-rabbit IgG (Cappel) as the secondary antibody, color development was carried out by using Konica Immunostain HRP1000. The samples used in lanes 1, 2 and 3 were immunoprecipitation products by the GAH antibody of the tumor cell, and the samples used in lanes 4, 5 and 6 were immunoprecipitation products by the GAH antibody of cultured cell. Lanes 1 and 4 were detected by Vectastain Elite ABC, lanes 2 and 5 were detected by ant-nmMHC antibody, and lanes 3 and 6 were detected by normal rabbit IgG.

Bands were detected on the lanes 1, 2 and 5.

Based on this result, it was found that the protein of about 200 kd, namely nmMHCA, is recognized by the GAH antibody in both of the cultured cell and the tumor cell, and nmMHCA is present on the cell surface in the case of the tumor cell.

Example 2

Confirmation using nmMHCA forced expression cell line:

Preparation of nmMHCA expression vector

HA1.0 and HALES (obtained from Robert S. Adelstein) as the nmMHCA gene were digested with a restriction enzyme *Eco*RI (Takara Bio). On the other hand, a plasmid vector pEF1/Myc-HisB for mammalian cell gene expression (Invitrogen) into which the nmMHCA gene is to be introduced was also digested with the restriction enzyme *Eco*RI and then dephosphorylated. The nmMHCA gene fragments and pEF1/Myc-HisB fragments were ligated and transformed to obtain a sample into which HA1.0 was introduced (named pEF1B-HA1.0) and a sample into which HALES was introduced (named pEF1B-HALES). A vector for full length nmMHCA mammalian cell expression was prepared by PCR (Advantage cDNA PCR kit, Clontech) using pEF1B-HA1.0 as the template and using the primer of SEQ ID NO: 18 in the Sequence Listing and the primer of SEQ ID NO:19 in the Sequence Listing. This PCR product was named *Kpn*I-HA1.0-*Spe*I. On the other hand, pEF1B-HALES was digested with restriction enzymes *Kpn*I and *Spe*I (both Takara Bio). The *Kpn*I-HA1.0-*Spe*I was inserted into the pEF1B-HALES restriction enzyme digest and then transformation of *Escherichia coli* was carried out. By carrying out mapping of plasmids of the thus obtained clones, it was confirmed that the vector of interest for full length nmMHCA mammalian cell expression was prepared.

Preparation of COS-7 forced expression cell line

The nmMHCA gene was introduced into the plasmid vector pEF1/Myc-HisB for mammalian cell gene expression (Invitrogen), and gene transfer into a *Cercopithecus aetiopus* kidney-derived cultured cell line COS-7 cell (obtained from Cell Resource Center for Biomedical Research, Institute of Development, Aging and Cancer, Tohoku University) was carried out by lipofection using PolyFect (Qiagen). The gene-transferred cells were cultured at 37°C in the presence of 5% $CO_2$ and used after 48 hours in the immunoprecipitation test as transient expression cell lines. After the gene transfer, a stable expression cell line was established by culturing at 37°C in the presence of 5% $CO_2$, followed by drug selection of a stable expression cell line using Genetecin G418 (Sigma). In addition, for the purpose of taking influences of gene transfer operation and drug selection operation into consideration in carrying out tests using the nmMHCA stable expression cell line, a mock cell of COS-7 was prepared as the negative control. The mock cell was prepared by carrying out the drug selection after transferring the gene into COS-7 cell by lipofection using a plasmid moiety (pEFI/myc-HisB) alone of the plasmid used in integrating the nmMHCA gene.

Preparation of HCT-15 stable expression cell line

Preparation of an nmMHCA gene-introduced stable expression cell line was carried out in the same manner as the case of COS-7, using a human colon cancer cell line HCT-15 cell (obtained from Cell Resource Center for Biomedical Research, Institute of Development, Aging and Cancer, Tohoku University). The mock cell was prepared also in the case of HCT-15.

Immunoprecipitation by GAH antibody

Each of the nmMHCA transient expression cells and un-introduced cells of COS-7 and HCT-15 were recovered by using a scraper, and 0.5 ml of a solubilization buffer was added thereto, followed by ultrasonic treatment (output 2, frequency 50%) for 5 seconds to disrupt the cells. After allowing to stand on ice for 1 hour, they were centrifuged at 15,000 rpm for 10 minutes using a microtube centrifuge to obtain a solubilized supernatant. In order to make protein concentrations contained in the thus obtained supernatants uniform, determination of protein was carried out by using BCA Protein Assay Kit (Pierce) in the same manner as in Example 1. Immunoprecipitation was also carried out in the same manner as in Example 1, and the immunoprecipitation products and solubilized supernatants for comparison were subjected to SDS-PAGE (gel concentration 6%) and then to Western blotting. The membrane was incubated at room temperature for 1 hour in a blocking buffer (PBS containing 0.1% gelatin and 0.05% Tween 20, 0.05% sodium azide (Wako Pure Chemical Industries)), and the anti-nmMHC antibody was diluted 100 times with the blocking buffer and allowed to react at room temperature for 1 hour. After the reaction, it was washed 3 times with PBST (PBS containing 0.05% Tween 20) at room temperature for 5 minutes and allowed to react at room temperature for 1 hour with an anti-rabbit IgG-HRP-labeled antibody which had been diluted 1,500 times with an HRP-labeled substance dilution buffer (PBS containing 0.1% gelatin). After the reaction, it was washed 3 times with PBST at room temperature for 5 minutes, and then bands were detected by using an luminescence substrate ECL (Amersham). The samples used in lanes 1, 2 and 3 are nmMHCA-introduced cells, and the lane 1 is a solubilized supernatant, lane 2 is an immunoprecipitation product by GAH antibody, and lane 3 is an immunoprecipitation product by human Igs. Also, the samples used in lanes 4, 5 and 6 are mock cells, and the lane 4 is a solubilized supernatant, lane 5 is an immunoprecipitation product by GAH antibody, and lane 6 is an immunoprecipitation product by human Igs.

A band of about 200 Kd was found in the lanes 1 and 2.

It was found from this result that nmMHCA is GAH antibody-specifically immunoprecipitated only in the nmMHCA transient expression cell line.

Confirmation for Preparation of nmMHCA stable expression cell line by Western blotting

Each of the stable expression cells after drug selection and mock cells of COS-7 and HCT-15 were recovered by using a scraper, and the SDS-PAGE sample buffer was added thereto, followed by ultrasonic treatment to disrupt the cells. Protein concentrations of the thus prepared samples were determined by using the BCA Protein Assay Kit, and they were subjected to SDS-PAGE (gel concentration 6%), Western blotting and detection by anti-nmMHC antibody.

While nmMHCA was equal to or lower than the detection limit in mock cell of COS-7, a band of about 200 kd corresponding to the molecular weight of nmMHCA was found in the nmMHCA-stably introduced cell line FL11. Also, in the case of HCT-15, while nmMHCA was equal to or lower than the detection limit in mock cell, nmMHCA was detected in the nmMHCA-stably expressing cell lines FL1 and FL2, and it was shown that the expressed amount was large in comparison with FL1.

Immuno-staining of nude mouse transplantation cancer cell sections using nmMHCA-expressing cell lines

Various nmMHCA-stably expressing cell lines of HCT-15 were suspended in MatriGel (Becton Dickinson) and subcutaneously transplanted into 2 spots under the back of a nude mouse at a dose of $5 \times 10^6$ cells/spot. Tumor was extracted after confirming its development, and a part thereof was soaked in 10% formalin-PBS to prepare paraffin sections, followed by hematoxylin-eosin (HE) staining at Nara Pathology Institute.

Each section was treated with xylene and ethanol to remove paraffin, soaked in 10 mM sodium citrate, pH 6.0 buffer, exposed to microwave (3 times, 600 W 5 minutes for each), air-cooled by allowing to stand at room temperature for 30 minutes, and then soaked in 5% (w/v) BSA-PBSAz solution for 1 hour. The section was incubated with 66 µg/ml of biotin-labeled $F(ab')_2$ fragment of GAH antibody, or 100 µg/ml of biotin-labeled anti-nmMHC antibody, at 37°C for 2 hours, and then allowed to react with 2.5 times diluted streptoavidin PerCP (Becton Dickinson) under ice-cooling and shade. After the reaction, red fluorescence of PerCP in the same visual field of each section was observed using Olympus incident-light fluorescence microscope BX-50.

The results are shown in Fig. 2. In comparison with the tissue section of mock cell (Mock) used as the control, GAH antibody showed strong red fluorescence for the tissue sections derived from the nmMHCA-stably expressing cell lines (FL1, FL2, FL7). Also, regarding anti-nmMHC antibody, distinct red fluorescence was observed only in the tissue section derived from the nmMHCA-stably expressing cell lines. In addition, the red fluorescence image originated from anti-nmMHC antibody was similar to the red fluorescence image originated from GAH antibody.

It was shown from this result that reactivity of GAH in tissue sections is increased by the introduction of nmMHCA.

Reactivity of GAH antibody with intact cells using nmMHCA-stably expressing cell line

An nmMHCA-stably expressing cell line was suspended in MatriGel and subcutaneously transplanted into 2 spots under the back of a nude mouse at a dose of $5 \times 10^6$ cells/spot. Tumor was extracted after confirming its development and cut into thin pieces to collect tumor cells. FITC-labeled GAH antibody or FITC-labeled human immunoglobulin was diluted with human serum (Scanty Bodies Laboratory) to a concentration of 33 µg/ml. Also, FITC-labeled anti-nmMHC antibody or FITC-labeled rabbit IgG was diluted with human serum to a concentration of 50 µg/ml. These antibody solutions were thoroughly mixed with each of tumor cells and allowed to react for 1 hour under shade and ice-cooling. After completion of the reaction, the cells were washed with PBS containing 0.1% sodium azide, and their flow cytometry analysis was carried out in the following manner by using Becton Dickinson BD-LSR. Cells were suspended in FACSflow (buffer attached to the flow cytometer) solution containing 5 µg/ml PI, PI-positive cells, namely dead cells, were excluded from the subject of analysis by gating operation in the same manner as in Example 1, and the mean value of FITC fluorescence intensity per one cell in the intact cell population was calculated. A calibration curve was prepared from the mean value, measured under the same condition, of fluorescence intensity of standard fluorescent beads (Flow Cytometry Standards) wherein the number of bound FITC molecules is already known, and the mean value of each sample was converted into the amount of bound FITC. The thus obtained value was further divided by the F/P value of each FITC-labeled antibody and used as the antibody binding number.

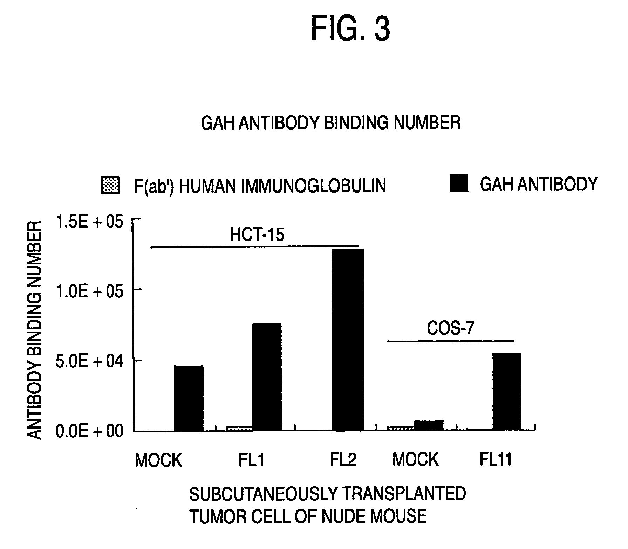

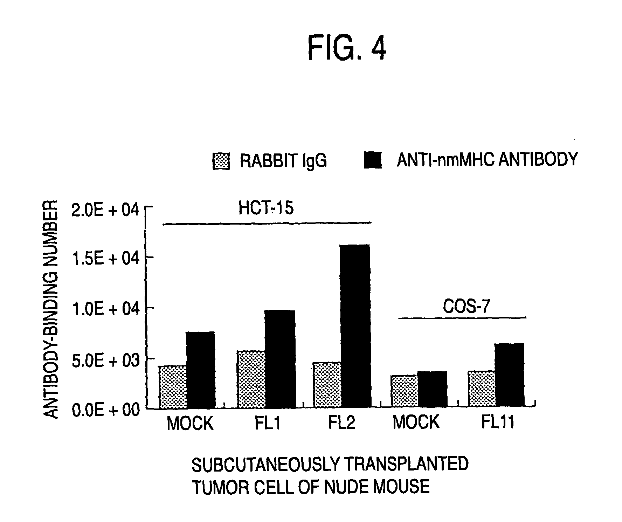

The results are shown in Fig. 3 and Fig. 4. It was shown that the GAH reactivity is increased in the nmMHCA-stably expressing cell lines of both COS-7 and HCT-15, in comparison with the mock cell lines.

It was shown from this result that reactivity of GAH upon the surface of transplanted tumor cells is increased by introducing nmMHCA.

Example 3

Confirmation of cell surface reactivity by anti-nmMHCA peptide antibody:

The peptides of SEQ ID NOs:20, 21 and 22 in the Sequence Listing as partial peptide sequences of nmMHCA were synthesized and linked to Keyhole Limpet Haemocyanin (KLH), and rabbits were immunized therewith to prepare polyclonal antibodies for respective peptides. Each of the antibodies was purified by using an affinity column in which the corresponding peptide was immobilized on CNBr Sepharose (Amersham Bioscience). Each of the purified polyclonal antibodies and normal rabbit immunoglobulin (normal rabbit IgG: Biogenesis) for control were labeled with FITC, and each antibody was prepared into 50 μg/ml and allowed to react, at 4°C for 1 hour, with cultured cells of MKN45 and tumor cells formed by subcutaneous transplantation of MKN45. After washing once with PBS, analysis was carried out in PBS containing PI using a flow cytometer (LSR: Becton Dickinson). PI-positive cells, namely dead cells, were excluded from the subject of analysis by gating operation. By calculating the mean channel number (mean value) of FL1 as an index of FITC fluorescence intensity, comparison of respective antibodies was carried out.

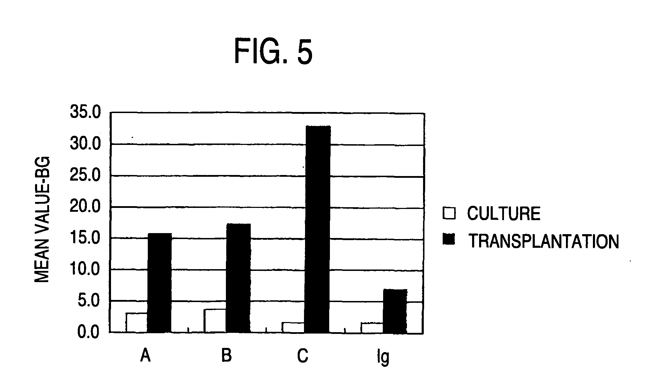

The results are shown in Fig. 5. A, B and C indicate respective anti-nmMHCA peptide antibodies, and Ig indicates normal rabbit immunoglobulin for control. The ordinate shows a value obtained by subtracting a background value (BG) in the case of cells alone from the mean channel number (mean value). In the explanatory notes in the drawing, "culture" indicates MKN45 cultured cells, and "transplantation" indicates tumor cells formed by the transplantation of MKN45.

When the increasing degree of reaction by each antibody in the tumor cells formed by the transplantation of MKN45, in comparison with the MKN45 cultured cells, was calculated by subtracting the reaction of normal rabbit immunoglobulin for control as a background value, 3 times or more of increase was found by the antibody A and antibody B, and 10 times or more of increase by the antibody C.

Based on this result, it was found that, among nmMHCA peptides, the partial sequences represented by SEQ ID NOs:20, 21 and 22 in the Sequence Listing are locally present on the cell surface. In addition, it was found that the existing amounts of the partial sequences represented by SEQ ID NOs:20, 21 and 22 in the Sequence Listing are increased in the tumor cells formed by the transplantation of MKN45 in comparison with the MKN45 cultured cells.

Example 4

Confirmation of antigen amount and antitumor activity:

Cancer cell cultured cell lines

The human colon cancer cell lines Caco-2, DLD-1 and SW620 were obtained from American Type Culture Collection. The human colon cancer cell line WiDr-Tc and the human esophageal cancer cell line TE-8 were obtained from Cell Resource Center for Biomedical Research, Institute of Development, Aging and Cancer, Tohoku University. The human gastric cancer cell lines HSC-3, MKN-1 and MKN45 and the human colon cancer cell line SW837 were obtained from Immuno-Biological Laboratories. The B37 cell line was established from a human gastric cancer by a conventionally known method.

Measurement of antigen amount on the surface of cancer cells

Subcutaneous tumor transplantation-derived cancer cells of various cancer cell lines were prepared in accordance with the method described in Example 1. Reaction quantities for various cancer cells were detected by flow cytometry in accordance with the method of Example 1, except that $F(ab')_2$ fragment of GAH antibody was labeled with FITC, and the reaction was carried out on ice for 1 hour by adjusting concentration of the $F(ab')_2$ fragment of GAH antibody to 50 μg/ml. In addition, in order to express the fluorescence detection quantity as antibody reaction quantity (antigen amount) per cancer cell, a fluorescence latex (Flow Cytometry Standard) having known FITC content was used as the standard in the determination.

Preparation of immuno-liposome and liposome

Using the F(ab')$_2$ fragment of GAH antibody, an antibody-linked liposome (immuno-liposome) was prepared in accordance with the method of EP-A-1174126. That is, a doxorubicin (DXR)-enclosed immuno-liposome was formed using a lipid mixture consisting of dipalmitoylphpsphatidylcholine/cholesterol/$\varepsilon$-maleimidocaproyldipalmitoylphpsphati-dylethenolamine (18/10/0.5 in molar ratio), a polyethylene glycol derivative (PEG) having two polyethylene glycol chains described in the same publication and the F(ab')$_2$ fragment of GAH antibody. Particle diameter of the thus obtained immuno-liposome was from 125 nm to 160 nm, and the quantity ratio of F(ab')$_2$ fragment of GAH antibody/PEG/DXR/lipid was 0.2 : 0.8 : 1 : 10 (weight ratio).

Correlation between antigen amount and *in vivo* antitumor activity

Each of various cancer cells was subcutaneously transplanted into nude mouse to effect formation of a tumor mass. The tumor mass was cut into small pieces of several mm$^3$ and transplanted under the skin membrane of the kidney of another nude mouse [Bennette *et al*., 1985, *Cancer Res*., 45, 4963-4969 (1985)]. Starting on the next day, DXR and immuno-liposome were administered from caudal vein (3 mg/kg as DXR) every other week, 3 times in total. The animal was dissected on the 22nd day, and weight of the extracted tumor was measured. A physiological saline-administered group was used as the negative control. As an index of the effect, tumor growth inhibition ratio was calculated based on the following formula.

$$\text{Tumor growth inhibition ratio (\%)} =$$

$$(1 - \text{mean tumor weight of drug-treated group} / \text{mean tumor weight of}$$

$$\text{negative control group}) \times 100$$

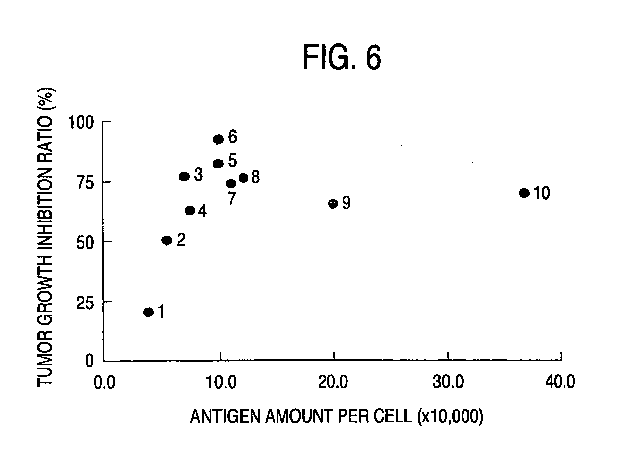

The results are shown in Fig. 6. The ordinate shows the tumor growth inhibition ratio (%), and the abscissa shows the antigen amount per cancer cell. In the drawing, reference numerals represent the following cancer cell lines. 1: Caco-2; 2: DLD-1; 3: HSC-3; 4: SW620; 5: SW837; 6: MKN-1; 7: B-37; 8: MKN45; 9: TE-8; 10: WiDr-Tc.
As a result, it was shown that the tumor growth inhibition ratio was improved as the antigen amount increases on cancer cells having an antigen amount per cancer cell of approximately up to 10$^5$/cell, and the antitumor effect reached almost plateau on cancer cells showing an antigen amount of more than that.

Example 5

Binding activity test on F(ab')$_2$ fragment of GAH antibody:

Preparation of MKN45-immobilized plate

MKN45 cell line was inoculated into a flask containing a culture medium and cultured in a CO$_2$ incubator. When plate face of the flask became a state of being filled with the cells, the culture medium was discarded, and the cells were peeled off by adding a trypsin solution (2.5 g of trypsin 1:250 (Difco) 0.2 g of Na$_2$EDTA (Sigma) were dissolved with PBS and adjusted to 1,000 ml) and transferred into a centrifugal tube. After centrifugation, the supernatant was removed and the cells were suspended in fresh culture medium. After counting the number of cells, they were suspended in the culture medium to a density of about 4 $\times$ 10$^5$ cells/ml, added at 100 μl to each well of 96 well plate and then cultured for 2 days. The culture medium in wells was discarded, PBS was added at 200 μl to each well, and the liquid was discarded. Next, a p-formaldehyde solution was added at 150 μl to each well and allowed to stand still at room temperature for 1 hour, and then the p-formaldehyde solution in the wells was discarded. An operation in which a PBS solution (Sigma) was added bit by bit in each well and then the liquid was discarded was carried out 5 times. The PBS solution (Sigma) was added at 200 μl to each well, and the plate was stored at 4°C.

Preparation of standard solutions and sample solutions

Standard solutions 1 to 6 were prepared by adding a dilution solution obtained by diluting 1 g of bovine serum albumin (Sigma) with PBS to a standard stock solution containing $1 \times 10^6$ units/ml of the $F(ab')_2$ fragment of GAH antibody. Also, sample solutions 1 to 6 having protein concentrations equivalent to the standard solutions were prepared using the above dilution solution in accordance with the protein content of each sample solution.

| Standard solutions | Concentration (unit/ml) | Sample solutions | Concentration (ng/ml) |
|---|---|---|---|
| Standard solution 1 | 1000 | Sample solution 1 | 1000 |
| Standard solution 2 | 500 | Sample solution 2 | 500 |
| Standard solution 3 | 250 | Sample solution 3 | 250 |
| Standard solution 4 | 125 | Sample solution 4 | 125 |
| Standard solution 5 | 62.5 | Sample solution 5 | 62.5 |
| Standard solution 6 | 31.25 | Sample solution 6 | 31.25 |

Operation method

An operation in which the liquid in wells of the plate was discarded, 200 μl of PBS is added to each well and then the liquid was discarded was carried out 5 times. Each of the standard solutions and sample solutions was added at 50 μl/well and allowed to stand still at 37°C for 2 hours, and then the liquid discarded. An operation in which 200 μl of PBS was added to each well and then the liquid was discarded was carried out 5 times.

Next, 50 μl of a horseradish peroxidase-labeled goat anti-human κ chain antibody solution (Cappel) was added to each well and allowed to stand still at 37°C for 1 hour. The liquid was discarded, 200 μl of a washing liquid was added to each well and allowed to stand still at 37°C for 5 to 10 minutes, and then the liquid was discarded. Subsequently, an operation in which 200 μl of the washing liquid was added to each well and then the liquid was discarded was carried out 5 times.

To each well, 100 μl of an o-phenylenediamine solution (Sigma) was added and allowed to stand still at room temperature for 1 to 2 minutes under shade. To each well, 100 μl of a stop solution was added and mixed by gentle shaking.

Using a microplate reader, absorbances A1 and A2 at wavelengths of 490 nm and 650 nm in each well were measured to calculate (A1 - A2).

A calibration was obtained by plotting the concentration of standard solutions as the abscissa and the absorbance (A1 - A2) as the ordinate. From the absorbance of each sample solution, titer per 1 ml of each sample solution (unit/ml) was obtained, and titer per 1 mg of the sample (unit/mg) was calculated.

As a result, it was found that the use of the $F(ab')_2$ fragment of GAH antibody having a binding activity titer of from 0.8 to $1.2 \times 10^6$ unit/mg is useful.

Industrial Applicability

According to the present invention, an antigen having a part which is exposed on the surface of a cell at the formation of a tumor mass can be provided.

Also, the present application was filed by claiming the priority of Japanese application No. 2002-291953, the entire contents of which are incorporated hereinto by reference.

## Claims

1. An antigen having a part which is exposed on the surface of a cell at the formation of a tumor mass.

2. The antigen according to claim 1, wherein the tumor mass is a solid tumor formed by subcutaneous transplantation of a cultured cancer cell.

3. The antigen according to claim 1 or 2, wherein the existing amount of the antigen of the solid tumor is increased in comparison with that of a cultured cell of the solid tumor.

4. The antigen according to any one of claims 1 to 3, wherein the existing amount of the antigen of the solid tumor on the cell surface is increased in comparison with that of a cultured cell of the solid tumor.

5. The antigen according to any one of claims 1 to 4, which is a cytoskeleton protein or a mutant thereof.

6. The antigen according to any one of claims 1 to 5, which is myosin or a mutant thereof.

7. The antigen according to any one of claims 1 to 6, which is a non-muscular myosin heavy chain type A or a mutant thereof.

8. The antigen according to any one of claims 1 to 7, which is a part of a non-muscular myosin heavy chain type A or a mutant thereof.

9. The antigen according to any one of claims 1 to 8, which is a sequence of a C-terminal domain of the protein sequence of a non-muscular myosin heavy chain type A or a mutant thereof.

10. The antigen according to claim 9, wherein the sequence of a C-terminal domain of the protein sequence is a sequence of the residue at position 600 to the residue at position 1,960 from the N-terminal of SEQ ID NO:17 in the Sequence Listing.

11. The antigen according to claim 9, wherein the sequence of a C-terminal domain of the protein sequence is any one of SEQ ID NOs:20, 21 and 22.

12. A ligand which recognizes the antigen according to any one of claims 1 to 11.

13. The ligand according to claim 12, which is an antibody.

14. The ligand according to claim 12 or 13, which is a monoclonal antibody.

15. The ligand according to any one of claims 12 to 14, wherein the monoclonal antibody is a human monoclonal antibody.

16. The ligand according to any one of claims 12 to 15, which is a cancer reactive monoclonal antibody.

17. The ligand according to claim 16, wherein the cancer is gastric cancer, breast cancer, colon cancer or esophageal cancer.

18. The ligand according to any one of claims 12 to 17, wherein a heavy chain hypervariable region comprises the amino acid sequences of SEQ ID NOs:1, 2 and 3 in the Sequence Listing, and a light chain hypervariable region comprises the amino acid sequences of SEQ ID NOs:4, 5 and 6 in the Sequence Listing.

19. The ligand according to any one of claims 12 to 18, which comprises a heavy chain variable region comprising the amino acid sequence of SEQ ID NO:7 in the Sequence Listing and a light chain variable region containing the amino acid sequence of SEQ ID NO:8 in the Sequence Listing.

20. A pharmaceutical composition, which comprises the ligand according to any one of claims 12 to 19.

21. The pharmaceutical composition according to claim 20, which is a targeting therapy agent.

22. The pharmaceutical composition according to claim 20 or 21, which targets at a cancer tissue or a cancer cell.

23. The pharmaceutical composition according to any one of claims 20 to 22, which comprises an antitumor agent, an antitumor protein, an enzyme, a gene or an isotope for treatment.

24. The pharmaceutical composition according to any one of claims 20 to 23, which is an antitumor agent.

25. The pharmaceutical composition according to any one of claims 20 to 24, wherein the cancer is gastric cancer, breast cancer, colon cancer or esophageal cancer.

26. The pharmaceutical composition according to any one of claims 20 to 25, which comprises liposome.

27. A labeling agent, which comprises the ligand according to any one of claims 12 to 19.

28. The labeling agent according to claim 27, which specifically labels a cancer tissue or a cancer cell.

29. The labeling agent according to claim 27 or 28, wherein the cancer is gastric cancer, breast cancer, colon cancer or esophageal cancer.

30. The labeling agent according to any one of claims 27 to 29, which comprises a fluorescent, an enzyme, an isotope or an MRI contrast medium.

**31.** A method for treating a cancer disease of a cancer disease patient which expresses the antigen according to any one of claims 1 to 11, which comprises administering the pharmaceutical composition according to any one of claims 20 to 26.

**32.** A method for treating a cancer disease of a cancer disease patient having a cell which can be labeled by the labeling agent according to any one of claims 27 to 30, which comprises administering the pharmaceutical composition according to any one of claims 20 to 26.

**33.** The ligand according to any one of claims 12 to 19, wherein the binding activity of the ligand which recognizes the antigen according to any one of claims 1 to 11 to the antigen is from $0.5 \times 10^6$ units/mg to $2.0 \times 10^6$ units/mg.

**34.** The ligand according to any one of claims 12 to 19, wherein the binding activity is from $0.7 \times 10^6$ units/mg to $1.5 \times 10^6$ units/mg, from $0.7 \times 10^6$ units/mg to $1.3 \times 10^6$ units/mg, or from $0.8 \times 10^6$ units/mg to $1.2 \times 10^6$ units/mg.

**35.** The ligand according to any one of claims 12 to 19, wherein the binding activity is from $0.8 \times 10^6$ units/mg to $1.2 \times 10^6$ units/mg.

# FIG. 1

# FIG. 2

# FIG. 3

## GAH ANTIBODY BINDING NUMBER

▒ F(ab') HUMAN IMMUNOGLOBULIN    ■ GAH ANTIBODY

# FIG. 4

# FIG. 5

FIG. 6

## INTERNATIONAL SEARCH REPORT

| International application No. |
| --- |
| PCT/JP03/12732 |

**A. CLASSIFICATION OF SUBJECT MATTER**
Int.Cl⁷ C07K14/705, C07K16/28, A61K9/127, A61K39/395, A61K51/00,
A61P35/00, C12N15/09

According to International Patent Classification (IPC) or to both national classification and IPC

**B. FIELDS SEARCHED**

Minimum documentation searched (classification system followed by classification symbols)
Int.Cl⁷ C07K14/705, C07K16/28, A61K9/127, A61K39/395, A61K51/00,
A61P35/00, C12N15/09

Documentation searched other than minimum documentation to the extent that such documents are included in the fields searched

Electronic data base consulted during the international search (name of data base and, where practicable, search terms used)
JSTPlus(JOIS), MEDLINE(STN), BIOSIS(STN), CAS(STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>A | EP 520499 A1 (Mitsubishi Kasei Corp.),<br>30 December, 1992 (30.12.92),<br>& US 5767246 A    & JP 5-304987 A<br>& CA 2072249 A | 12-30,33-35<br>1-11 |
| X | EP 399257 A1 (OREGON HEALTH SCI. U.),<br>28 November, 1990 (28.11.90),<br>& US 5200508 A    & JP 3-81299 A<br>& CA 2015862 A | 1-4,12-16,<br>20,24,33-35 |
| X | MODAK Shakeel et al., 'Monoclonal Antibody 8H9<br>Targets a Novel Cell Surface Antigen Expressed<br>by a Wide Spectrum of Human Solid Tumor', CANCER<br>RESEARCH 61, 4048-4054, (2001) | 1,3,4,12-16,<br>33-35 |

☒ Further documents are listed in the continuation of Box C.   ☐ See patent family annex.

| * Special categories of cited documents: | "T" later document published after the international filing date or |
| --- | --- |
| "A" document defining the general state of the art which is not considered to be of particular relevance | priority date and not in conflict with the application but cited to understand the principle or theory underlying the invention |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 13 November, 2003 (13.11.03) | 02 December, 2003 (02.12.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)

## INTERNATIONAL SEARCH REPORT

| | International application No. |
|---|---|
| | PCT/JP03/12732 |

C (Continuation). DOCUMENTS CONSIDERED TO BE RELEVANT

| Category* | Citation of document, with indication, where appropriate, of the relevant passages | Relevant to claim No. |
|---|---|---|
| X | WO 92/08131 A1 (UNIV. COLUMBIA NEW YORK),<br>14 May, 1992 (14.05.92),<br>& US 6159751 A    & EP 641385 A1<br>& JP 6-505147 A | 1,3,4,12-17,<br>20-30,33-35 |
| X | WO 02/057741 A2 (MOLECULAR DISCOVERIES, L.L.C.),<br>25 July, 2002 (25.07.02),<br>& US 2002/137109 A1    & EP 1350085 A2 | 1,3,4,12-17,<br>20-30,33-35 |
| X | DIPPOLD Wofgang G. et al., 'A Common Epithelial<br>Cell Surface Antigen(EPM-1) on Gastrointestinal<br>Tumors and in Human Sera', CANCER RESEARCH 47,<br>3873-3879, (1987) | 1,3,4,12-16,<br>33-35 |
| X | OHUCHI Noriaki et al., 'Characterization of Cell<br>Surface Antigens Expressed in the HMA-1 Breast<br>Cancer Cell Line', Surg.Today 25, 244-250, (1995) | 1,3,4,12-16,<br>33-35 |
| X | IMAM S.A. et al., 'Identification of a Cell-<br>Surface Antigen (LEA.135) Associated with<br>Favorable Prognosis in Human Breast Cancer',<br>CANCER RESEARCH 53, 3233-3236, (1993) | 1,3,4 |
| A | NATALI Pier G. et al., 'Heterogeneous distribution<br>of actin, myosin, fibronectin and basement membrane<br>antigens in primary and metastatic human breast<br>cancer', Virchows Archiv A: Pathological Anatomy<br>and Histopathology, 405(1)69-83, (1984) | 5-11 |

Form PCT/ISA/210 (continuation of second sheet) (July 1998)

<table>
<tr><td>**INTERNATIONAL SEARCH REPORT**</td><td>International application No.<br>PCT/JP03/12732</td></tr>
</table>

**Box I   Observations where certain claims were found unsearchable (Continuation of item 2 of first sheet)**

This international search report has not been established in respect of certain claims under Article 17(2)(a) for the following reasons:

1. ☒ Claims Nos.: 31, 32

because they relate to subject matter not required to be searched by this Authority, namely:
The inventions as set forth in claims 31, 32 involve methods for treatment of the human body by therapy and diagnostic methods and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest.

☐ No protest accompanied the payment of additional search fees.

Form PCT/ISA/210 (continuation of first sheet (1)) (July 1998)

35